# EUROPEAN PATENT APPLICATION

(11) **EP 3 878 349 A1**
(43) Date of publication of application: **15.09.2021**
(21) Application number: 20162475.6
(22) Date of filing: 11.03.2020
(51) Int. Cl.: A61B 1/005, A61B 1/018, A61B 1/05

(54) **MODULAR FLEXIBLE ENDOSCOPE**

(71) Applicant: Stichting Katholieke Universiteit, 6525 EZ Nijmegen (NL)
(72) Inventor: Verhoeven, Roel Lambertus Johannes, 6525 GA Nijmegen (NL); van der Heijden, Henricus Franciscus Maria, 6525 GA Nijmegen (NL)
(74) Representative: Nederlandsch Octrooibureau

(57) **Abstract**

A medical system comprises a catheter (100) comprising a flexible tubular structure, the catheter having a lumen (106) extending in longitudinal direction through the catheter (100), wherein a material of a segment (102) of the catheter is more flexible with respect to bending than a remainder of the catheter. A connector (601, 901) can attach the first elongated structure (600) to the second elongated structure (900) near the distal end (605) of the first elongated structure (600) and the distal end (905) of the second elongated element (900). The first elongated structure (600) and the second elongated structure (900) have a cross section (608, 907) configured to together removably fit through the lumen (106) of the catheter (100). A proximal portion of the first elongated structure (600) up to the connector (601) and a proximal portion of the second elongated structure (900) up to the connector (901) are movable.

## Description

### FIELD OF THE INVENTION

The invention relates to a medical system. The invention relates in particular to a medical system for intraluminal imaging. The invention relates in particular to a medical system for intraluminal interventions. The invention further relates to a medical system for endoscopy. The invention further relates to a medical system for endobronchial endoscopic imaging and interventions.

### BACKGROUND OF THE INVENTION

Small peripheral pulmonary lesions suspected of lung cancer or any other early stage cancer leading to small abnormalities in organs with confined endoluminal spaces remain a diagnostic challenge. Small peripheral pulmonary lesions are increasingly often incidentally diagnosed as a consequence of, for example, a CT scan requested for another co-morbidity. As the resolution of CT and the ease with which they are requested has increased, more nodules are being found. Meanwhile, computed tomography screening programs are being implemented globally and will increase the number of detected peripheral pulmonary lesions suspected of early stage lung cancer. Once detected on imaging, these peripheral pulmonary lesions need to be further evaluated and/or diagnosed. The preferred methodology to obtain a diagnosis or treat these lesions suspected of lung cancer is to be as minimally invasive as possible. Yet, with the currently available technology, lesions in the outer segments of the lung are hard, if not impossible, to reach by minimally invasive tools in a safe, fast and effective manner.

Several tools and techniques have been designed over the past years to overcome these shortcomings. Examples of techniques that envision an endobronchial approach for navigating towards and inspecting these nodules include, for example, radial ultrasound mini probe imaging, ultrathin bronchoscopy, virtual navigation bronchoscopy, robotic bronchoscopy, cone beam computed tomography intra-operative guidance and augmented fluoroscopy, artificial intelligence multi-modal platforms, and electromagnetic navigation bronchoscopy.

The pulmonologist is faced with having to combine multiple technologies for enhancing diagnostic yield. Whereas they were previously only familiar using separate technologies, i.e. CT imaging, fluoroscopy for global localization, endobronchial ultrasound (EBUS), or video-endoscopic imaging, it is now expected that they are able to combine all technologies at once. At the same time, the pulmonologist sees more difficult clinical cases increasingly often. An indication for lesion biopsy is lowered to even the smallest nodes, necessitating the most tortuous navigation and most accurate biopsy positioning. Similar problems may occur in other medical intraluminal and endoscopic procedures, such as endovascular, urogenital, or gastro-intestinal endoscopic procedures.

It would be desirable to have a more cost-effective technology or a technology that is more easy to deploy. Moreover, it would be desirable to provide a technique that can be used to perform most functions that are needed throughout the procedure.

United States Patent 5,243,967 discloses an endoscope system that comprises an endoscope to be inserted into body cavities, a light source for supplying illumination light, an air and water, and suction unit for supplying air and water, and sucking up contaminants from body cavities, a motor control unit for driving and controlling a motor for bending, a camera control unit for processing video signals originating from a solid-state imaging device in the endoscope, a monitor for displaying video signals, a VTR for recording the video signals, and a remote control for remotely operating the motor control unit. A motor control unit control circuit in the motor control unit and a camera control unit control circuit in the camera control unit communicate with each other for mutual control.

### SUMMARY OF THE INVENTION

It would be advantageous to have an improved medical system for intraluminal procedures. To address this concern, a medical system is provided that comprises
a catheter comprising a flexible tubular structure, the catheter having a lumen extending in longitudinal direction through the tubular structure of the catheter, wherein a material of a segment of the catheter is more flexible with respect to bending than a remainder of the catheter;
a first elongated structure and a second elongated structure;
a connector for attaching a distal end of the first elongated structure to a distal end of the second elongated structure;
the first elongated structure and the second elongated structure having a cross section configured to together removably fit through the lumen of the catheter;
a proximal portion of the first elongated structure up to the connector and a proximal portion of the second elongated structure up to the connector being movable with respect to each other at least in a longitudinal direction of the first elongated structure and second elongated structure.

The medical system may be highly suitable to navigate the catheter to the desired position within a relatively narrow lumen. The bending of the more flexible segment can be controlled by manipulating the first elongated element and the second elongated element from a proximal position, when the two elongated structures are attached to each other and are within the catheter and overlapping the more flexible segment. Since the first elongated structure and the second elongated structure are removable from the lumen of the catheter, these structures may be removed from the lumen when the catheter has been correctly positioned, leaving the catheter in place. Thereafter, another suitable tool may be inserted through the catheter to be guided by the catheter to the desired place at the distal end of the catheter. This way, the catheter can be relatively thin, because it only needs to contain either the first and second structures, or the tool, but not all of the first and second structures and the tool at the same time.

The second elongated structure may comprise a lumen extending from a proximal end of the second elongated structure to the distal end of the second elongated structure, the lumen being suitable to allow passage of a third elongated structure. The lumen of the second elongated structure may be used, for example, to bring a small tool or a guidewire to the distal end. When retracting the second elongated structure from the catheter, the third elongated structure may be left behind, so that another tool may be inserted through the catheter around the guidewire for better guidance. Alternatively, other kinds of tools may be forwarded through the lumen of the second elongated structure.

The first elongated structure may comprise at least one detector at its distal end, wherein the detector comprises for example a camera, an optic fiber, an ultrasound imaging probe, or a radiation monitoring probe (such as a gamma probe). The first elongated structure can thus be used as an endoscope or a means of imaging and sensing. Since the first elongated structure does not need to have an open lumen, the first elongated structure may be easy to clean, facilitating re-use of the expensive detector. For example, the detector may be implemented by a sensor, such as a semiconductor, e.g. a CMOS or CCD chip. Alternatively, the detector may be implemented as an optic fiber to relay rays incident on the detector onto a sensor positioned in a more proximal location. The camera may be a visible light camera, an infrared camera, a near-infrared camera, an ultraviolet camera, a multispectral camera, or any other kind of camera. To enhance the imaging result of e.g. the camera, the first elongated structure may further comprise a source of illumination, for example a lamp, such as a light emitting diode, which may be coupled to the distal end of the first elongated structure by means of an optic fiber. The first elongated structure may further comprise a means of detecting a radiation source, such as a gamma probe.

The connector may be configured for detachably fixing the distal end of the first elongated structure to the distal end of the second elongated structure. This way, the two elongated structures can be detached after use, for example after having been retracted from the catheter. This further facilitates cleaning and allows to dispose, for example, the second elongated structure while re-using the first elongated structure. Alternatively, it may allow to continue to use the second elongated structure during a procedure, together with another type of first elongated structure. The other type of first elongated structure may comprise, for example, a different type of sensor.

The second elongated structure may comprise a needle that is configured to be actuated proximally and, when actuated, move at least partially out of the distal end of the second elongated structure to puncture a tissue. This may facilitate cell aspiration or the guiding of a third elongated structure to be inserted into the tissue, for example through an optional lumen of the needle. The needle may be made of, for example, nitinol, a metal, or any other suitable material. The needle may be hollow.

The medical system may comprise the third elongated structure, the third elongated structure having a cross section configured to fit through the lumen of the second elongated structure and optionally through a lumen of a needle at a distal end of the first or second elongated structure. This allows easy provisioning of a thin elongated structure to the desired position.

The catheter may be configured to cooperate with a tissue piercing distal part of a fourth elongated structure, wherein the catheter is configured to, when activated whilst the fourth elongated structure is in place, cut tissue along a circumference of the lumen of the tubular structure of the catheter when moved over the fourth elongated structure. This allows to cut and obtain a relatively large tissue specimen using a relatively narrow catheter. The catheter may be relatively narrow since it does not need to contain, at the same time, the first elongated structure and/or the second elongated structure together with the fourth elongated structure.

The medical system may comprise the fourth elongated structure, wherein the fourth elongated structure has a cross section configured to fit through the lumen of the catheter around the third elongated structure. Such a fourth elongated structure may be relatively easy to be guided to the desired location, since it may be guided by the third elongated structure.

The first elongated structure may have a first cross section and the second elongated structure may have a second cross section with an edge that matches a corresponding edge of the first cross section, wherein when the first elongated structure and the second elongated structure are joined at their matching edges the cross section of the combination of the first elongated structure and the second elongated structure have a substantially circular outline. This may facilitate the control of rotation of the combination of the first elongated structure and the second elongated structure around its longitudinal axis while the structures are in the catheter.

The cross section of the first elongated structure may be a circle segment and the cross section of the second elongated structure may be a circle segment. Such circle segments are a suitable example of the cross section with matching edges.

The more flexible segment of the catheter may be inclined to bend preferably in a predetermined direction. These features may further enhance control of the bending of the catheter. Alternatively, the more flexible segment of the catheter may be undirected, i.e. the more flexible segment may be equally flexible in all bending directions.

The more flexible segment of the catheter may be positioned at the distal end of the catheter. For example, the more flexible segment of the catheter may comprise the distal end of the catheter. This may further enhance control of the bending and the navigation of the catheter. For example, the catheter may be positioned at the distal end of the catheter, just proximal of a distal tip of the catheter that comprises a tool such as a cut tool.

The medical system may comprise an actuator arranged at a proximal end of the tubular structure of the catheter. The actuator may be configured to control longitudinal movement of at least one of the first elongated structure and the second elongated structure with respect to the other one of the first elongated structure and the second elongated structure, to achieve bending of the catheter at the flexible segment. This may further enhance control of the bending of the catheter.

According to another aspect of the invention, an endoscope is provided with a first endoluminal elongated structure comprising

At least one detector, such as a camera, an optic fiber, or an ultrasound probe, at a distal end of the elongated structure; and
a connector near the distal end of the first elongated structure for attaching a second elongated structure to the distal end of the first elongated structure.

This endoscope may be advantageously used to image and perform minimally invasive interventions, by attaching the connector to another elongated structure.

According to another aspect of the invention, a second endoluminal elongated structure may be provided, comprising
a lumen extending from a proximal end of the second elongated structure to a distal end of the second endoluminal elongated structure, the lumen being suitable to allow passage of a third elongated structure; and
a connector near the distal end of the second elongated structure for attaching a first elongated structure to the distal end of the second elongated structure.

This elongated structure may be advantageously used to perform minimally invasive interventions, by attaching the connector to another elongated structure.

The person skilled in the art will understand that the features described above may be combined in any way deemed useful. Moreover, modifications and variations described in respect of the system may likewise be applied to the method, and modifications and variations described in respect of the method may likewise be applied to the system.

### BRIEF DESCRIPTION OF THE DRAWINGS

In the following, aspects of the invention will be elucidated by means of examples, with reference to the drawings. The drawings are diagrammatic and may not be drawn to scale. Throughout the drawings, similar items may be marked with the same reference numerals.
Fig. 1 shows a frontal view of a catheter.
Fig. 2 shows a right view of the catheter.
Fig. 3 shows a back view of the catheter.
Fig. 4 shows a left view of the catheter.
Fig. 5 shows a top view of the catheter.
Fig. 6 shows a view of a first example of a first elongated structure from a left/frontal direction.
Fig. 7A shows a top view of the first elongated structure.
Fig. 7B shows a diagram of the cross section of the first elongated structure.
Fig. 8 shows a right view of the first elongated structure.
Fig. 9 shows a view of a first example of a second elongated structure from a back/left side.
Fig. 10 shows the second elongated structure from the back.
Fig. 11A shows a top view of the second elongated structure.
Fig. 11B shows a diagram of a cross section of the second elongated structure.
Fig. 12 shows a top-left view of a second example of a second elongated structure.
Fig. 13 shows a top view of the second elongated structure.
Fig. 14 shows a first example of a third elongated structure.
Fig. 15 shows a left view of a second example of a third elongated structure.
Fig. 16 shows a front view of the third elongated structure.
Fig. 17 shows a left view of a second example of a first elongated structure.
Fig. 18 shows a front view of the first elongated structure.
Fig. 19 shows a right view of the first elongated structure.
Fig. 20 shows a back view of the first elongated structure.
Fig. 21 shows a frontal view of a fourth elongated structure.
Fig. 22 shows an enlarged detail of Fig. 21.
Fig. 23 shows a frontal/top view of the fourth elongated structure.
Fig. 24 shows a top view of the fourth elongated structure.
Fig. 25 shows a flowchart illustrating a method of exploring a lumen.

### DETAILED DESCRIPTION OF EMBODIMENTS

Certain exemplary embodiments will be described in greater detail, with reference to the accompanying drawings.

The matters disclosed in the description, such as detailed construction and elements, are provided to assist in a comprehensive understanding of the exemplary embodiments. Accordingly, it is apparent that the exemplary embodiments can be carried out without those specifically defined matters. Also, well-known operations or structures are not described in detail, since they would obscure the description with unnecessary detail.

The procedural approach for endoluminal procedures can often be divided into two main steps: The first objective may be to navigate to an abnormality accurately. However, in targeting peripheral lung lesions, easy access by means of navigating through the natural lumen of the bronchial tree is not possible or difficult at least. This may also apply to other endoluminal targets. The second step may involve obtaining tissue. In diagnosis of abnormalities in the periphery of the lung, usage of tools can both be endoluminal (but very often these cannot be visualized by a detector due to the location being unreachable by videoscope) and trans-parenchymal. Tool usage is then commonly blind (i.e. on the basis of memory) or under a secondary external imaging source, such as C-arm imaging, intra-operative CT, or MRI imaging). Similar problems may occur for other endoluminal targets.

As a lesion in the lung may commonly be of about a cubic centimeter in size and often has complex relations when related to the remainder of its environment and the bronchial tree, accurate navigation is a complex task. While identification of the lobe and segment containing the lesion may be achieved based on i.e. pre-procedural CT, further intra-procedural navigation is not easy. With every sub-segmental and consecutive branching of the bronchial tree (with high inter-patient anatomical variation), a wrong turn is easily made and orientation easily lost. Using existing technology, the nodule and the route with which a catheter or device is directed towards said nodule is furthermore very often not easily visualized entirely or is often not visible endo-bronchially at all, as it might be accessible only trans-parenchymal, being located too far peripheral for conventional imaging with video-endoscopy or ultrasound (as currently known to the field) or have only very limited surface area adjacent to the bronchial wall. As every nodule is unique, there is no standardization in this process.

Navigation guidance, to increase likelihood of procedural success, is currently available to the field using multiple methodologies. Classically, a conventional bronchoscope could be used for these procedures. From the working channel of the conventional bronchoscope, tissue/cell acquisition tools were then directly extended. This could be either completely blind (or based on memory from CT review) or with help of a catheter as extended through the working channel of the endoscope and/or fluoroscopic guidance (i.e. X-ray through C-arm fluoroscopy).

Over the last decade, several additional techniques have become available that can help guide navigation to the peripheral lesion in combination with the use of an endoscope. Examples are given in the following.

Radial endobronchial ultrasound (EBUS) mini probe imaging as extended through the working channel for intermittent imaging and biopsy. While small in size and usable for reaching the outer peripheral of the lung, limitations of this technology are the intermittence with which it needs to be used (due to no working channel being available for secondary tools if introduced) and the limited imaging capabilities. The side viewing radial scanning imaging characteristics make it difficult to see if lesions are in front of the probe or at which orientation they are. If the lesion is not directly adjacent to the bronchus it can furthermore not be visualized using the R-EBUS mini probe (as the lungs contain air).

Virtual bronchoscopy navigation, wherein a pre-procedural CT is combined with the real-time videoscopic image for navigation guidance. The depth to which one can navigate relies on pre- and intra-procedural CT scan quality and endoscope diameter. Video-endoscope diameter herein is often the main limiting factor. This limitation mainly lies in the endoscopes as known to the field having a working channel for biopsy tool guidance and this needing to be sufficiently big for specimen acquisition.

Fluoroscopy based guidance has historically been known, but has regained interest with the possibility of smart recombination of imaging. Where it was previously used only as coarse method for directional confirmation of endoscope and catheter, it is now used in combination with more advanced systems. These systems for example use pre-procedural CT imaging information for registration of fluoroscopy to CT. With a limited resolution, these are then for example able to overlay bronchial tree anatomy and lesion positioning onto the fluoroscopic image and help navigate. It can however not accurately (with sub-centimeter accuracy) compensate in 3D for motion. Due to motion (respiratory/cardiac/endoscope) and x-ray imaging being performed from the outside (global imaging) and in 2D, margin of error is quite large and navigation can be cumbersome.

Electromagnetic navigation technology, wherein a weak electromagnetic field enables tracking of one or multiple sensors within the endoscope or endoscopic tools. It is based on pre-procedural or intra-operative CT imaging, and can possibly be further updated with fluoroscopic imaging. It can provide guidance on navigation but also biopsy location when it is integrated within the biopsy tool. As it is not cross-verified with real-time information and has limited resolution, it again leaves room for error. Often, it needs usage of additional tools for navigating to outer segments. This reduces intuition, and does not give real-time confirmation of lesion positioning.

Intra-operative Cone beam CT imaging is available only for a limited number of centers due to associated expense. It enables intra-procedural imaging and confirmation in 3D of lesion and tool positioning. Recombination with real-time fluoroscopic imaging further enables lesion augmentation on fluoroscopic imaging. However, this technique also leaves room for error due to e.g. movement of a patient.

In ultrathin bronchoscopy, a bronchoscope with limited size and fixed working channel is used to navigate to the peripherals. Such an ultrathin bronchoscope has only a small working channel. This implies that only very small biopsy samples can be taken. To a certain extent, ultrathin bronchoscopy may allow for navigation towards the peripherals of the lungs. However, due to the working channel that is needed for the biopsy, an ultrathin bronchoscope is still of bigger size than what would be ideal. Moreover, the working channel is relatively narrow. Therefore, such a device may have a problem to guide biopsy specimen acquisition modalities of size. Moreover, treatment using certain secondary devices (e.g. flexible microwave ablation probes) remains impossible.

Robotic endoscopy may involve a robot/motor actuation to replace the manual manipulation of the endoscope by the pulmonologist. Technologies include ultrathin video-endoscopes, or endoscopes housing a secondary working channel, shape sensing technology, motorized actuation and navigation guidance based on virtual navigation bronchoscopy and/or electromagnetic tracking. Such robots use conventional endoscopic biopsy tools/methodologies, with known limitations. The integration of robotic actuation at the distal tip of the steerable endoscope further means that the working channel through which tools are inserted are of lesser diameter. The technology of the robot is associated with significant complexity and cost.

Flexible endoscopic tools for diagnosis of pathology, have been used for decades. Their purpose are all similar: obtaining tissue (histology) or cells in order to enable a diagnosis. Hollow needles may be used for aspirations (cytology). Alternatively, forceps may be used. A cryoprobe works by freezing the distal tip to an extent such that it tightly freezes adjacent (superficial) tissue. By traumatic extraction of the device with adjacent tissue, one obtains tissue for histopathology analysis.

As the lesion may be of a cubic centimeter size and may be situated peripherally, direct video-endoscopic visualization is often not feasible with current technology. Moreover, not all of the volume of the lesion may contain viable tumor tissue for diagnosis. Making the procedure more difficult is that these nodules can often have complex relations when related to the remainder of its environment and the tools used. The lesion is furthermore often stiffer than surrounding tissue. Tool insertion may more easily deform surrounding tissue and also aspirate/biopsy surrounding tissue, especially if a pushing technique for tissue acquisition is used. Lesion positioning relative to the tool is very often not such that every biopsy direction will acquire suspected pathological tissue along the biopsy tool insertion direction. The nodule is often of small size, making the exact insertion position of the tool very important. Tool insertion (trans-parenchymal) and biopsy is often performed under no local vision (i.e. only C-arm imaging for global positioning, with limited resolution). Tool insertion is done on basis of prior knowledge rather than real-time knowledge. Stiffness of differently used endoscopic tools will affect tissue positioning. As a consequence of these factors, multiple biopsies may be needed for obtaining an adequate and representative diagnosis. This may involve using several different tools for obtaining tissue specimens in the hope of obtaining an adequate diagnosis.

Not alone the discrepancy between navigation and diagnostic success shows the potential value which can be added by a more suitable biopsy and navigation tool, the needed time and amount of different tools used further prove this. All considering, the demand for an intuitive, safe, fast and accurate minimally invasive (endobronchial and transparenchymal) navigation guidance, biopsy, and potentially treatment enabling toolset for peripheral pulmonary lesions is high and is anticipated to even increase as screening programs for early stage lung cancer are being initiated. Other fields where flexible endoscopy in confined spaces is subject of interest, such as in urology and gastroenterology, are another clinical field of interest to which the techniques disclosed herein may be applied.

Several components are disclosed herein. These components may be able to integrate with one another and combined enable a safe and cost-effective medical workflow and combination of functionalities. Certain embodiments allow to make maximal use of device dimensions in confined spaces by integrative design. The techniques disclosed herein may enable an intuitive workflow in endoscopic imaging, biopsy and/or treatment in confined spaces. The individual components disclosed herein have certain functionality. The integration of several of these components with each other may enable additional functionality which is not available when the components are used alone.

The techniques disclosed herein may provide better uses of available lumen sizes and/or allow smaller diameter devices to be used to navigate through a relatively small lumen and/or take a biopsy from within such a small lumen. Further, certain embodiments may provide advantages with respect to efficient cleaning and/or efficient re-use of components.

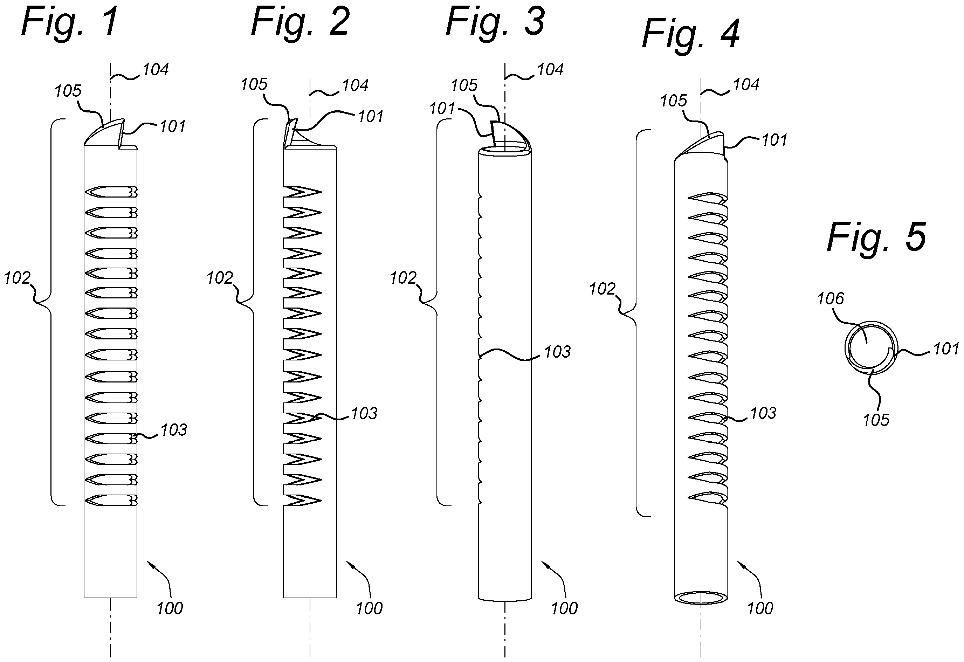

Fig. 1 through Fig. 5 show different views of an example of a distal end of a catheter. Assuming the catheter tip is held up vertically, Fig. 1 shows a frontal view of the catheter. Fig. 2 shows a right view of the catheter. Fig. 3 shows a back view of the catheter. Fig. 4 shows a left view of the catheter. Fig. 5 shows a top view of the catheter, showing the distal tip as seen from a distal position beyond the distal tip of the catheter.

The catheter may be made of any material known in the art for catheter production. Moreover, the diameter of the catheter may be relatively small. In certain examples, the catheter has an outer diameter of at most three millimeters. The diameter of the lumen may be less than, for example, 2.4 millimeters in such examples. The wall of the catheter may have a thickness of submillimeter range. However, it is emphasized that the principles and advantages of the present disclosure may be implemented using components of any suitable dimensions, both bigger and smaller.

In the shown implementation, the catheter has a cutter 101 at the distal tip. The cutter is configured to cut tissue when the catheter is rotated along its longitudinal axis 104 in a counterclockwise direction when viewing the catheter in a direction from proximal towards distal. Of course, this is not a limitation. The catheter may also be configured to cut when rotated in the other direction. In an alternative embodiment, the catheter may have a movable component that enables cutting by actuation. Moreover, the cutter 101 is optional.

The catheter may alternatively be made without cutter 101. In case a biopsy is desired, another tool may be inserted through the catheter to actually cut the tissue.

Whether or not a cutter is provided, the distal tip 105 may be atraumatic in nature if a material comes into contact with said distal tip 105 along the insertion/retraction direction of the catheter 100, such as to prevent damage to other devices, tools, or tissue if it were to be introduced or retracted inside or through another device or a lumen in tissue.

The cutter 101 of the distal tip 105 may be atraumatic in nature if a second material outside the tubular lumen comes into contact with said distal tip, when no secondary component is inserted into the tubular lumen. Such to prevent damage to other devices/tools/tissue if it were to be introduced or retracted inside or through said devices or endoluminal space. For example, the cutter 101 may be configured to cooperate with another tool to perform a cutting operation when the other tool is moved with respect to the cutter 101, similar to two blades of a pair of scissors.

For example, tissue cutting may be realized by moving the catheter 100 with respect to another component protruding from the catheter lumen 106 at the distal end 105 of the catheter 100.

In the embodiment shown in the drawings, the cutter 101 is configured to cut tissue if the catheter 100 is rotated around its longitudinal axis 104 in a first direction. Moreover, the cutter 101 is configured to be atraumatic if the catheter 100 is rotated around its longitudinal axis 104 in a second direction opposite to the first direction.

In an alternative embodiment, the cutter 101 may be configured to cut tissue if it were rotated, regardless of whether the rotation is in the first or second direction. This may be realized, for example, by a cutter with two blades, one for each direction.

In yet another alternative embodiment, the cutter 101 may be configured to cut tissue if it were pushed over a secondary component that may be present inside and protrude from the lumen 106 of the catheter 100.

In the shown implementation, the cutter 101 protrudes from the wall of the catheter at the distal end of the catheter and comprises a cutting blade oriented parallel to or slightly inclined with respect to the longitudinal axis 104 of the catheter 100. The cutting blade may be inclined slightly forward and towards the central axis 104 of the catheter 100 in order to prevent inadvertent cutting while moving the catheter in distal direction along its longitudinal axis 104.

The shown catheter 100 further comprises a segment 102 that is more flexible with respect to bending than a remainder of the catheter 100. Specifically, the catheter has slits 103 oriented substantially perpendicular to the longitudinal axis 104 of the catheter 100. This way, the catheter segment 102 is biased to bend in the direction where the slits are, i.e. in the forward direction. The substantially perpendicular orientation of the slits 103 with respect to the longitudinal axis 104 of the catheter, may help to keep the flexible segment 102 stiff with respect to torsion around the longitudinal axis.

Alternative embodiments may employ different techniques to realize the segment with increased flexibility. For example, the segment 102 may be made of a different material. Alternatively, the segment 102 may have a thinner wall thickness than the remainder of the catheter. It is noted that the segment 102 may be biased towards a specific bending direction, but this is not a limitation. It is also possible that the segment 102 is simply more bendable in all directions.

In certain embodiments, there may be more flexible segments similar to segment 102, along the catheter. However, these are not illustrated in the drawing.

In certain embodiments, a (distal) portion of the catheter 100 may be fluoroscopically opaque, or contain one or more fluoroscopically opaque markers. This may facilitate fluoroscopic guidance or provide additional fluoroscopic information.

In certain embodiments, a (distal) portion of the catheter 100 may be electromagnetically trackable, either from outside or by an electromagnetic sensor that may be removably inserted in to the catheter 100 through its lumen 106.

The catheter may extend a certain length in the proximal direction (not shown), opposite the distal end 105.

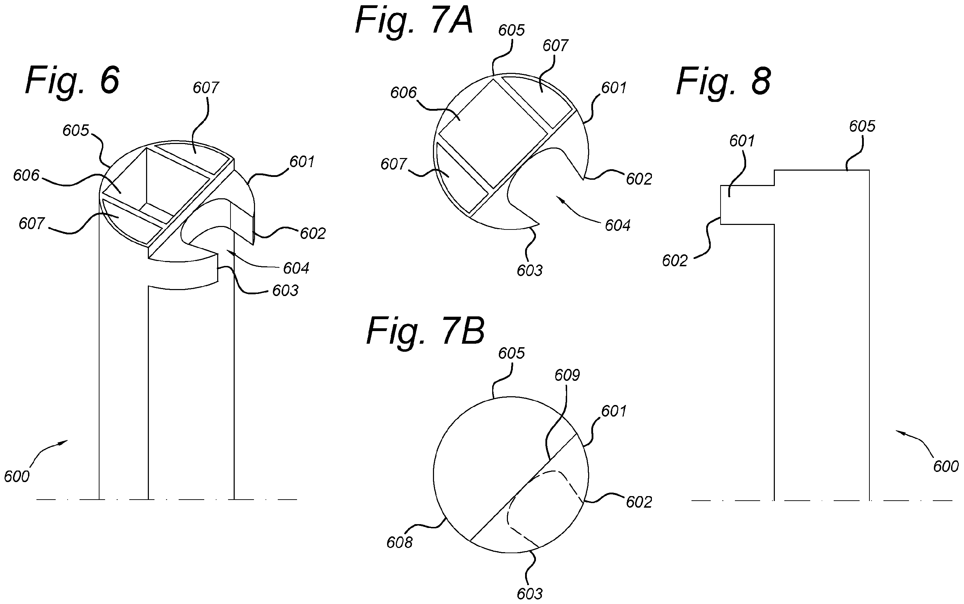

Fig. 6 through Fig. 8 show a first example of a first elongated structure 600. Fig. 6 shows a view of the first elongated structure 600 from a left/frontal direction. Fig. 7A shows a top view of the first elongated structure 600. Fig. 7B shows a diagram of the cross section of the first elongated structure 600. Fig. 8 shows a right view of the first elongated structure 600.

The first elongated structure comprises a connector 601 at the distal end 605 of the first elongated structure 600. The connector 601 is configured to connect to another object, in particular another elongated structure.

In the embodiment shown, the connector 601 is implemented as two protruding elements 602, 603, that can click and hold an element that fits into a space 604 in between the two protruding elements 602, 603. However, this is not a limitation. Any connector 601 that can connect to a reciprocating object may be implemented as the connector 601.

It is observed that the first elongated structure 600 may be equipped with any kind of tool or tools, such as any kind of detector or means of imaging and detecting. In the illustrated example, a detector 606 is disposed at the distal end 605. The detector 606 is an optic fiber that transmits radiation incident on the tip of the optic fiber to a more proximal location, where a camera (not illustrated) is fitted. The camera may be sensitive to visible light or any other suitable spectrum of radiation, including near infrared, infrared, and/or ultraviolet. Alternatively, the camera may be disposed directly at the distal end 605 of the first elongated structure, omitting the optic fiber.

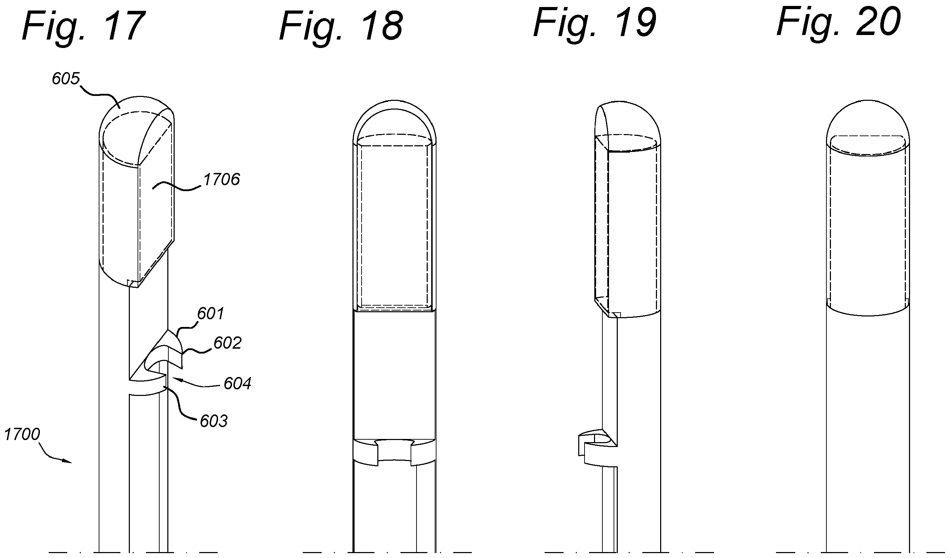

In certain embodiments, the detector 606 may comprise an ultrasound probe 1706 instead of, or in addition with a camera. This is described in greater detail elsewhere in this disclosure, with reference to Fig. 17. The detector 606 may comprise any other suitable kind of detector instead of, or in addition to, a camera, optic fiber, and/or ultrasound probe. An example of such another kind of detector is a radiation monitoring probe, such as a gamma probe.

Further, an illumination system 607, which may comprise for example one or more light emitting diodes (LEDs) or another type of lamp, may be provided at the distal end 605 of the first elongated structure 600. This illumination system 607 may enhance visibility of objects to be detected by the detector 606. The illumination system 607 and/or detector 605 may be powered by electricity using an electricity cable (not illustrated) extending from proximal end to the camera and/or illumination system 607 at the distal end 605, inside the first elongated structure 600.

As may be best appreciated from Fig. 7A and Fig. 7B, the cross section 608 of the first elongated structure 600, excluding the connector 601, may be regarded to have the shape of a circle segment. In Fig. 7B, the cross section 608 is illustrated in a solid line, and the connector 601 is illustrated in a dashed line. The connector 601 is disposed on the edge of the circle segment. This way, the connector 601 is configured to fix another structure to the edge 609 of the circle segment 608. This feature allows two components to form a substantially circular cross section together.

Differently shaped cross sections may be envisaged, wherein, for example, the cross section of the first elongated structure has the shape of a circle wherein a portion of the circle is removed, and the connector is configured to fix another structure into this removed portion of the circle.

The first elongated structure 600 may be able to provide a video-endoscopic image, as known to the (flexible) endoscopic field per se.

The first elongated structure 600 may comprise an imaging modality known to the field per se, enabling video or image capture at the distal tip 605 of the first elongated structure 600, and relaying said image to the proximal end of the first elongated structure 600 or, wirelessly, to a remote receiver. Common examples include CMOS chips, CCD chips or optical relay based systems such as optical fibers.

The illumination system 607 may be implemented as any illumination system known by itself in the field of endoscopy, to provide illumination at the distal tip of the first elongated structure. Possible methods of illumination for example include LEDs at the distal tip of the component, or, fiber-based relay of light from a proximal position to the distal tip 605.

The first elongated structure 600 may have any arbitrary geometry, safeguarded from the external environment. The design of the proximal end may be such that it facilitates usage in combination with other components, disclosed herein, and handling in an ergonomic way. The first elongated structure 600, including its distal tip 605, may be flexible to enable and retain steering capability of guiding devices or flexible endoscopes when placed in or outside the device. The design of the first elongated structure 600 may be such that it can be incorporated in a secondary tubular hollow structure, e.g. the catheter 100, or any component or device, that has a diameter greater than the outer diameter. The cross section of the first elongated structure may have an arbitrary geometry, while facilitating insertion into the lumen 106 of the catheter 100. The distal end 605 of the first elongated structure may contain a structure that facilitates introduction into a hollow tubular component in a guided fashion. The distal end 605 of the first elongated structure 600 may contain a structure, e.g. the connector 601, that can be used for mounting the distal tip of a second elongated structure.

The first elongated structure 600 may be constructed without any enclosed working channels or hollow structures that are in fluid communication with the environment external to the first elongated structure 600. This feature may facilitate cleaning and reusability. Indeed, problems of cleaning and/or mechanical defects may be reduced by omitting a working channel in the first elongated structure.

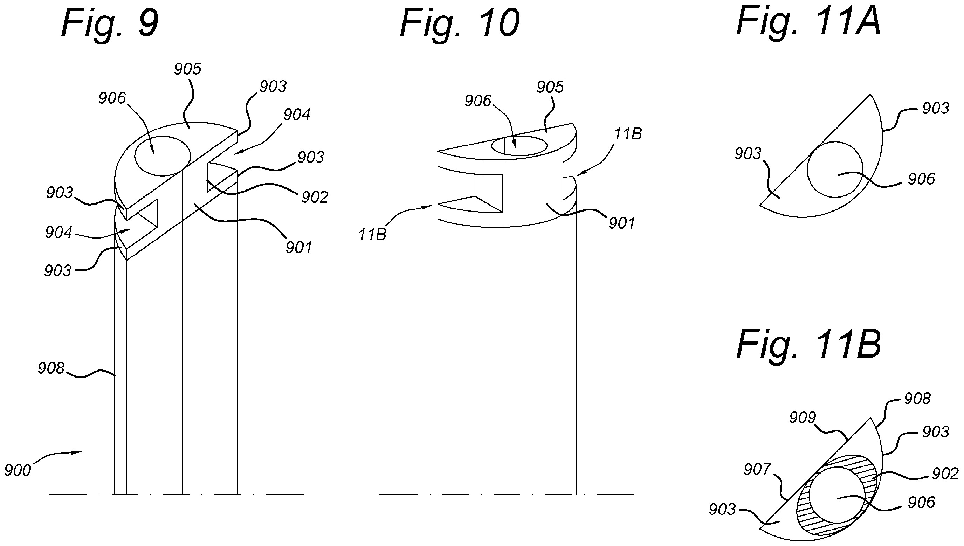

Fig. 9 to Fig. 11 show a first example of a second elongated structure 900. Fig. 9 shows a view of the second elongated structure 900 from a back/left side. Fig. 10 shows the second elongated structure 900 from the back. Fig. 11A shows a top view of the second elongated structure 900. Fig. 11B shows a diagram of a cross section 907 of the second elongated structure 900.

The second elongated structure 900 has a connector 901 at the distal end 905.

In the example shown, the connector 901 is configured to cooperate with the connector 601 of the first elongated structure 600. The connector 901, in the example shown, comprises a core 902 that can click into the space 604 in between the two protruding elements 602, 603 of the connector of the first elongated structure 600. Blocking elements 903 embrace spaces 904 that can receive the protruding elements 602, 603. These blocking elements 903 may prevent longitudinal movement of the first elongated structure 600 with respect to the second elongated structure 900 at the distal ends 605, 905 thereof, by blocking the protruding elements 602, 603 in the longitudinal direction.

It will be understood that the implementation of the connector is provided purely as an example. Other types of connector, such as other clicking mechanisms may be used for the purpose of fixing the first and second elongated elements 600, 900 together near their distal ends 605, 905. The skilled person will be able to apply a known type of connector, in view of on the present disclosure. Preferably, the connector is implemented in such a way that the first and second elongated structures can be detached from each other after use. In the clicking connector example shown in Figs. 6 through 11, detachment may be realized by pulling the two distal ends 605, 905 away from each other.

The medical system, formed by the catheter 100, the first elongated structure 600, and the second elongated structure 900, with the first elongated structure 600 connected to the second elongated structure 900 at the distal end and the combined first elongated structure and second elongated structure having been inserted into the lumen of the catheter 100 so that the first and second elongated structure overlap the more flexible segment 102 of the catheter, may be used to control bending of the medical system at the more flexible segment 102. This bending may be controlled by moving the second elongated element 900 with respect to the first elongated element 600. This movement may be controlled from a proximal position, by hand or by means of an actuator (not illustrated). Since the first and second elongated structure are fixed to each other at the distal end, the bending occurs as a consequence of the movement.

For example, the first elongated structure 600 and the second elongated structure 900 may be movable with respect to each other at least in a longitudinal direction, except at the fixed distal end 605, 905 of the first elongated structure and second elongated structure.

For example, the proximal portion of the first elongated structure 600 from the proximal end (not illustrated) of the first elongated structure 600 up to the connector 601 and a proximal portion of the second elongated structure 900 from the proximal end (not illustrated) of the second elongated structure 900 up to the connector 901 may be movable with respect to each other at least in a longitudinal direction of the first elongated structure and second elongated structure. Alternatively, at least a portion of the first elongated structure 600 and second elongated structure 900 between a proximal actuator or controller (not illustrated) and the connector 601, 901 may be movable with respect to each other.

The second elongated structure 900 may comprise an optional lumen 906, extending from the distal tip 905 towards a proximal location, such as the proximal end (not illustrated), of the second elongated structure 900. The lumen 906 may be used to insert another elongated structure through the lumen. This other elongated structure may be used to perform an action guided by the endoscope of the first elongated structure, for example.

As may be best appreciated from Fig. 11A and Fig. 11B, the cross section 907 of the first elongated structure 600, excluding the connector 601, may be regarded to have the shape of a circle segment. The circle segment of the cross section of the first elongated structure 600 may be complementary to the circle segment of the cross section of the second elongated structure 900, in the sense that together these cross sections form a circle.

In Fig. 11B, the cross section 907 of the second elongated structure along its length 908 is illustrated in a solid line, and the cross section of the connector core 902 is illustrated in a patterned surface, around the optional lumen 906. The connector 901 thus comprises two recesses of the second elongated structure, to receive the protruding elements 602, 603 of the first elongated structure 600. This way, the connector 901 is configured to fix to another structure around its core 902. This feature allows two components to form a substantially circular cross section together.

Differently shaped cross sections may be envisaged, wherein, for example, the cross section of the first elongated structure has the shape of a circle wherein a portion of the circle is removed, and the connector is configured to fix the second elongated structure in this removed portion of the circle, and such that together the two cross sections substantially fill the circle.

In alternative embodiments, other implementation of the connector may be used, preferably still resulting in a circular outline of the first and second elongated structures combined, although another outline, such as a parallelogram, square, or rectangle, may also be used instead of a circle as the shape of the outline. For example, the connector may comprise a magnetic connector to replace the click connector.

The proximal part (not illustrated) of the second elongated structure 900 may comprise an interface, e.g. an actuator, that can be used to exert a push force or a pull force along its longitudinal axis. The push force and/or pull force in combination with having fixed the two elongated structures together at their distal ends will enable a flexion of the medical system. The proximal end may facilitate usage in combination with the other components disclosed herein, and handling in an ergonomic way. The actuation can be mechanical or motorized or by other means of actuation known to the field per se. The distal end may be designed in one of many different ways to further enable guided and/or predictable introduction of tools or components into the distal endoscopic cavity. The second elongated structure 900 may be designed such to provide an additional (mechanical) safeguard for other components, such as the first elongated structure 600 or a biopsy or puncturing tool, used in conjunction with the second elongated structure 900, for example to prevent mechanical defects occurring by repeated use, or also, cleaning issues.

For example, the first elongated structure 600 and the second elongated structure 900 can be attached to one another at their distal tips 605, 905. After that, they can be inserted into the proximal end of the catheter 100, so that the distal ends of the first elongated structure 600 and the second elongated structure 900 are proceeded up to the distal end of the catheter 100, overlapping the flexible segment 102. By the combination of these three components 100, 600, and 900, steering and/or angulation of the device is enabled by pushing, pulling, or applying a force to a proximal portion of the second elongated structure 900, while the latter is fixed to the first elongated structure and retained within the catheter 100. The catheter 100 may comprise a flexible segment 102 near the distal tip 105. The flexible segment 102 may be biased to bending towards one or more specific sides, which bias may be used to improve control of the bending and steering the catheter 100. The footprint of the first elongated structure 600 may be aligned with the flexible segment 102 of the catheter 100, to enable easy and/or intuitive use. The footprint of the first elongated structure 600, perpendicular to the longitudinal axis, may have a shape and size that enables insertion into the catheter 100 and also, to enable insertion and guidance of the second elongated structure 900 in addition to the first elongated structure 600 into the endoscopic cavity in front of the distal tip 105.

The catheter, including the flexible segment, may be torsionally stiff. That is, when the catheter is rotated around its longitudinal axis at a proximal location of the catheter, the catheter tip will rotate along with the proximal portion of the catheter. This may allow to control the bending direction of the flexible segment. Moreover, it may allow to control an optional tool at the distal tip of the catheter.

A needle may be used in conjunction with the first elongated structure 600 and the second elongated structure 900 for providing a video-endoscopic guided puncture using a component such as a rigid guidewire or a small needle, such as a hollow aspirating needle. The imaging of the component by the detector 606, 1706 can help guide the needle or a similar tool for puncturing target tissue.

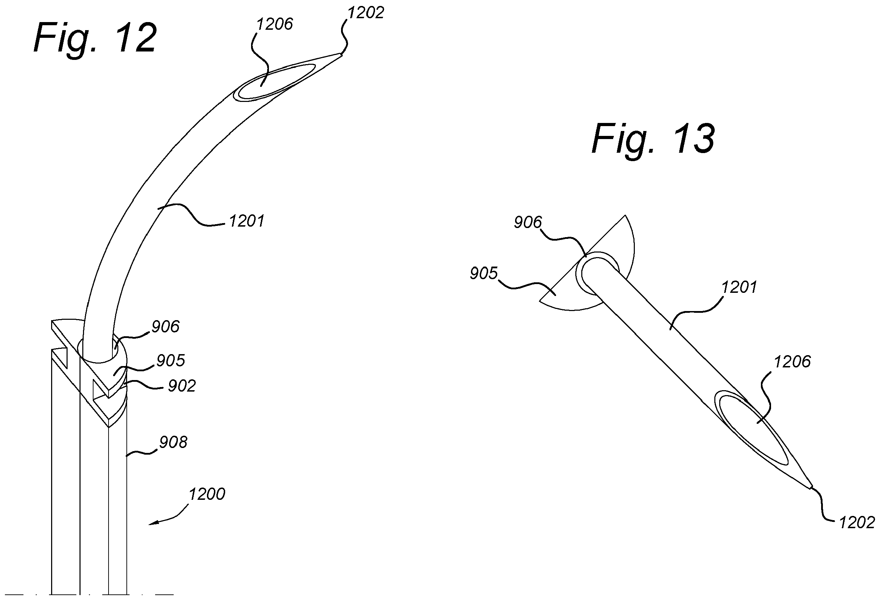

Fig. 12 and Fig. 13 illustrate a second example of the second elongated structure 1200 comprising an additional tool at the distal end 905. Fig. 12 shows a top-left view of the second elongated structure 1200, and Fig. 13 shows a top view of the second elongated structure 1200.

The second example of the second elongated structure 1200 comprises the same features as the first example of the second elongated structure 900. Therefore, these features will not be described again, and the same reference numerals may be used to indicate these features.

The second example of the second elongated structure 1200 comprises a needle 1201 having a lumen 1206 and a sharp tip 1202. The lumen 1206 is optional. The tip 1202 does not have to be sharp in all implementations. In the shown implementation, the lumen 1206 of the needle 1201 is fluidly connected with the lumen 906 of the main body 908 of the second elongated structure. These lumens are arranged coaxially, and allow another elongated structure to pass from a proximal end of the second elongated structure 1200 towards the distal end 905 and further into and through the lumen 1206 of the needle 1201 towards and beyond the tip 1202.

The needle 1201 may be movable with respect to the main body 908 of the second elongated structure 1200. Specifically, the needle 1201 may be retracted within the main body 908. Moreover, the needle 1201 may be moved from the retracted position to move out of the main body 908, beyond the distal end 906 of the second elongated structure 1200. This movement may be controlled from a proximal position, optionally using an actuator (not illustrated).

The needle 1201, may have a sharp tip 1202 in order to puncture a tissue.

Further, the needle 1201 may be biased to bend as it is moved out of the main body 908. For example, the needle 1201 may comprise a memory material. This makes it easier to puncture the tissue when the second elongated structure 1200 is inside a lumen of a tissue.

Instead of a needle, some other kind of puncturing and/or anchoring tool may be provided.

The distal tip 1202 of the needle 1201 may facilitate puncture of tissue. The component may be used in conjunction with the first elongated structure 600 for real-time guided tissue puncture (through e.g. ultrasound or video-endoscopic visualization). The needle 1201 may be made of a material with sufficient rigidity to enable a puncture of tissue. The component has sufficient flexibility to be used in flexible endoscopy, as understood by the skilled person in view of the present disclosure. The distal tip 1202 can, in certain embodiments (not illustrated) anchor and de-anchor itself in tissue once having punctured tissue, through expansion or retraction, by usage in combination with further devices through its lumen 1206, or, other techniques. When the needle 1201 has the lumen 1206, the needle may have an elongated hollow tubular structure, which can be utilized for guiding further devices. The needle 1201 itself may, in certain embodiments, contain technology for measuring tissue properties. The needle 1201 or lumen 906 may further comprise an integrated electromagnetic sensor, or, may be electromagnetically trackable or contain electromagnetically trackable sensors. The needle 1201 can have arbitrary geometry. The proximal end of the second elongated structure 1200 may facilitate usage in combination with the other components disclosed herein, and may facilitate handling in an ergonomic way.

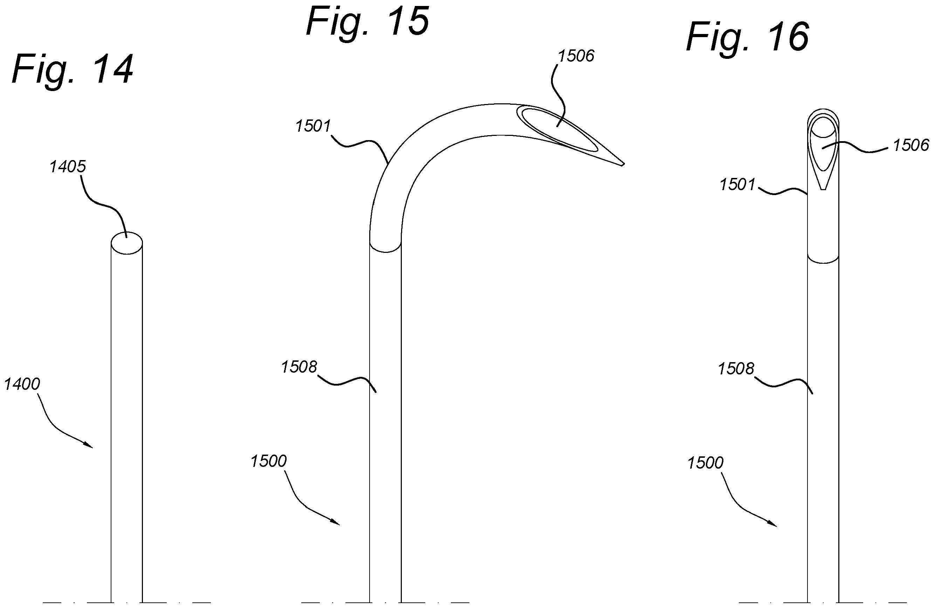

Fig. 14 shows a front view of a first example of a third elongated structure 1400. The third elongated structure has a distal tip 1405. The cross section of the third elongated structure may be small enough to fit through the lumen 906 of the second elongated structure 900, 1200 and optionally through the lumen 1206 of the needle 1201. For example, the third elongated structure 1400 is a guidewire.

In certain embodiments, while the needle 1201 has punctured the tissue, the third elongated structure 1400 is moved into the lumen 906, 1206. After that, the needle 1201 may be retracted as described above, leaving the third elongated structure 1400 behind in the tissue. After that, the whole of the first elongated structure 300 and the second elongated structure 1200 is removed from the catheter 100, leaving only the third elongated structure 1400 behind. Then, the third elongated structure 1400 can be used as a guidewire to insert another tool around the third elongated structure 1400 through the lumen of the catheter 100, optionally into the tissue.

Fig. 15 and Fig. 16 show a second example of the third elongated structure 1500. Fig. 15 shows a left view of the third elongated structure 1500. Fig. 16 shows a front view of the third elongated structure 1500. The third elongated structure 1500 comprises a main body 1508 and a needle 1501. The needle 1501 may be similar to the needle 1201. However, rather than being integrated into the second elongated structure, the needle 1501 is fitted to the distal end of the third elongated structure 1500. This allows the needle 1501 to be moved through the lumen 906 to puncture a tissue using the second elongated structure 900.

The example shown is a tubular embodiment. That is, a lumen 1506 extends through the third elongated structure 1500 up to the distal end of the needle 1501. This may allow a further elongated structure to be inserted through the third elongated structure 1500. However, this is not a limitation. The third elongated structure 1500 does not need to be hollow. For example, it can be used as a guidewire after the needle 1501 has punctured a tissue and the first elongated structure 600 and the second elongated structure 900 have been removed from the catheter 100.

The distal tip 1501 may facilitate easy puncture of tissue. The third elongated structure 1500 may be introduced through the lumen 906 of the second elongated structure 900. The third elongated structure 1500 may be used in conjunction with the first elongated structure 600 for real-time guided tissue puncture (through e.g. ultrasound or video-endoscopic visualization). The third elongated structure 1500 may be possibly introduced with use of the second elongated structure 900 for guided and/or predictable puncture and/or location properties once inserted into the catheter 100 proximally. The third elongated structure 1500, in particular the needle 1501, may have sufficient rigidity to enable a puncture of tissue distally by proximal insertion of the third elongated structure 1500 into the catheter 100. The third elongated structure 1500 may have sufficient flexibility to be used in flexible endoscopy, as understood by the skilled person in view of the present disclosure. The component can be used to further guide a tool for tissue specimen acquisition after having punctured / been deployed into the target tissue, as will be elaborated hereinafter. The distal tip 1506 of the needle 1501 can possibly anchor and/or de-anchor itself in tissue once having punctured tissue, through expansion or retraction, or, other techniques as known to the field per se. The third elongated structure 1500 may be of elongated hollow tubular structure, which can be utilized for guiding secondary devices. The third elongated structure 1500 may contain technology for measuring tissue properties. The third elongated structure 1500, in particular the needle 150, may be integrated with an electromagnetic sensor, or, may be electromagnetically trackable. Where elongated structure 1500 can have arbitrary geometry. The proximal end (not illustrated) of the third elongated structure 1500 may be such that it facilitates usage in combination with the other components disclosed herein, and may facilitate handling in an ergonomic way.

Fig. 17 through Fig. 20 illustrate a second example of the first elongated structure 1700. Fig. 17 shows a left view of the first elongated structure 1700. Fig. 18 shows a front view of the first elongated structure 1700. Fig. 19 shows a right view of the first elongated structure 1700. Fig. 20 shows a back view of the first elongated structure 1700.

The second example of the first elongated structure 1700 has many features in common with the first example of the first elongated structure 600. Therefore the description of these features is not repeated again. The same reference numerals may be used to indicate similar features. In particular features relating to the connector 601 and the cross section may be similar.

The second example of the first elongated structure 1700 differs from the first example 600 by a different type of detector. The first elongated structure 1700 has an ultrasound detector 1706 instead of an optic detector 606. It will be understood that in certain embodiments, both an optic detector and an ultrasound detector may be combined in a single first elongated structure (not illustrated).

The connector 601 of the second example of the first elongated structure 1700 is located near the distal end 605. However, the connector 601 is further away from the distal tip 605 in the second example of the first elongated structure 1700 compared to the first example of the first elongated structure 600. This is because it may be advantageous to protrude the ultrasound probe 1706 out of the catheter 100 to obtain better ultrasound signals, or to facilitate ultrasound guided actions, such as tissue puncture by the needle 1201 or guidewire 1500. Having the connector 601 a bit more proximal allows the second elongated structure 900, 1200 to be completely contained within the catheter 100 while the ultrasound probe 1706 protrudes from the catheter.

In general, the detector 606, 1706 may be in between the distal end 605 and the connector 601, regardless of the type of detector used. For example, the connector 601 may be adjacent the detector 606, 1706. When in operation to control bending of the catheter 100, the components may be positioned such that the connector 601, 901 is located distally with respect to the flexible segment 102 of the catheter 100. For example, the connector 601, 901 may be located at the distal end of the flexible segment 102 or more distal.

In the illustrated example, the ultrasound probe 1706 is directed laterally, so that it can create ultrasound images of lateral tissue. In an alternative embodiment (not illustrated), the ultrasound probe may be directed frontally so that an image of an object distal to the distal tip 605 of the first elongated structure 1700 is generated. In an alternative embodiment (not illustrated), the ultrasound probe may be designed radially so that an image of all tissue surrounding the probe may be obtained. In said alternative embodiment, the ultrasound probe may have a certain indication such that it can indicate the orientation of the probe with respect to the imaged tissue.

The ultrasonic elements located at the distal tip portion of the first elongated structure 1700, to whom electronics are provided within the first elongated structure 1700, may have an electric connection or a wireless connection connector to an external ultrasound processor / imaging device. The ultrasonic elements can be of piezo-electric origin, but also capacitive micro-machined ultrasonic transducer (CMUT) or piezoelectric micro-machined ultrasonic transducers (PMUT) origin, as will be understood by the person skilled in the art in view of the present disclosure. The ultrasonic elements can be mounted and/or electronically steered to be forward, oblique or radially (sideways) looking. The ultrasonic elements and their electronics may be incorporated in an elongated structure of arbitrary geometry, safeguarded from the external environment. Any imaging technology or software, as known to the person skilled in the art of ultrasonography, can be applied. The design of the proximal end (not illustrated) may facilitate usage in combination with the other components disclosed herein, and may facilitate handling in an ergonomic way. The first elongated structure 1700 and distal tip 605 may be flexible to enable and retain steering capability of guiding devices or flexible endoscopes when placed inside or outside the device.

The design of the component may be such that it can be incorporated in a secondary tubular hollow structure (component or device, e.g. catheter 100) that has a lumen with a diameter greater than the outer diameter. The elongated structure 1700 may be of arbitrary geometry perpendicular to its length, and may facilitate insertion into the catheter 100. The distal end of the first elongated structure 1700 may contain a structure facilitating introduction into a hollow tubular component in a guided fashion. The distal part of the first elongated structure 1700 may have a design enabling integration with the second elongated structure 900, 1200. The distal end of the first elongated structure 1700 may contain a structure that can be used for mounting the distal tip of a secondary component, such as the second elongated structure 900, 1200.

For example, the first elongated structure 1700 can be attached to the second elongated structure 900, 1200, using the connectors 601, 901 near the distal tips. After that, the elongated structures 1700, 900/1200 can be inserted into the proximal end of the catheter 100, so that the ultrasound probe 1706 protrudes from the distal tip 105 of the catheter 100. By the combination of these three components, proximal steering of the distal part of the catheter is enabled by pushing and/or pulling the proximal portion of the second (or the first) elongated structure. The catheter 100 may be designed specifically to enable flexibility of the distal portion to one or multiple sides, which is then used by the manipulating the first and second elongated structure to enable active steering. The footprint of the first elongated structure 1700 may align with the flexible part 102 of the catheter 100 to enable easy and intuitive use.

The footprint (cross section) perpendicular to the longitudinal direction of the first elongated structure may enable insertion into the catheter 100 and/or enable insertion and guidance of additional components, such as the second and/or third elongated structures or other tools into the endoscopic cavity as found at the distal tip 105.

The needle 1201, 1501 can be used in conjunction with the first and second elongated structure for providing an ultrasound guided puncture.

The first elongated structure 1700 may not have any enclosed working channels or structures that are in fluid communication with the environment external to the first elongated structure 1700, facilitating easy cleaning and re-usability of technology. The ultrasound elements may be positioned such to enable ultrasound imaging of tissue in an endoscopic cavity. The ultrasound elements may be positioned such to enable guidance of the needle 1201, 1501 or a similar component for guiding puncturing of target tissue. The obtained ultrasound image may be useful for component and lesion positioning, such to enable guided and/or registered manipulation of components.

The first example 600 and the second example 1700 of the first elongated structure may be integrated into a single elongated structure. For example, such a single elongated structure may comprise a video-endoscopic detector/optic fiber tip at the distal tip of the single elongated structure, and an ultrasound imaging component slightly proximal to the videoscopic detector/optic fiber tip.

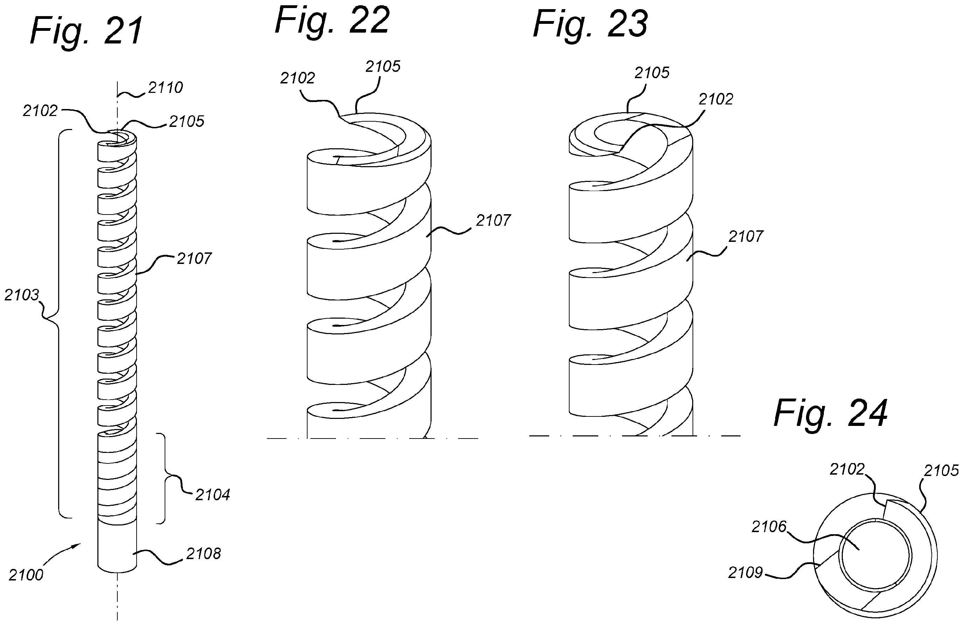

Fig. 21 through Fig. 24 show an example of a fourth elongated structure 2400. Fig. 21 shows a frontal view of the fourth elongated structure 2400. Fig. 22 shows an enlarged detail of Fig. 21. Fig. 23 shows a frontal/top view of the fourth elongated structure 2400. Fig. 24 shows a top view of the fourth elongated structure 2400.

The fourth elongated structure 2400 comprises a helical segment 2103 at the distal tip 2105. Moreover, the distal end of the helical segment may comprise a sharp puncturing tip 2102. The remainder of the fourth elongated structure (main body 2108) may be hollow or tubular. Preferably, the whole fourth elongated structure 2100 including the main body 2108 and the helical segment 2103 have a lumen 2106. In certain embodiments, the lumen 2106 can receive at least part of the third elongated structure 1400 or 1500.

Co-pending prior right European patent application No. 19205836.0, the contents of which is fully incorporated herein by reference, discloses a tissue biopsy device with a first tubular component and a second tubular component, the second tubular component surrounding the first tubular component. The first tubular component has a tissue piercing distal part with a hollow core, and the second tubular component has a distal end part with a cutting surface. The first tubular component and the second tubular component are flexible in a length wise direction, allowing to use the tissue biopsy device in a more versatile manner. The first tubular component of that application may be used as the catheter 100 of the present application. Moreover, the second tubular component of that application may be used as the fourth elongated structure of the present application.

The fourth elongated structure 2100 can fixate tissue by literally screwing the helical segment 2103 into the tissue. This screwing operation may be guided by the third elongated structure 1400 or 1500, which may have been inserted into the tissue beforehand, for example guided by the needle 1201 or 1501.

The outer diameter of the helical segment 2103 may be slightly smaller than the inner diameter of the catheter 100 (i.e., the diameter of the lumen of the catheter 100). This may facilitate smooth introduction of the segment 2103 into the flexible catheter. Moreover, it may help to retain concise cutting properties. Moreover, the cutter 101 may be positioned adjacent the circumference of the helical segment 2103 when the helical segment 2103 protrudes from the distal end 105 of the catheter.

The cutter 101 may be configured to cut tissue along a circumference of the helical segment 2103 when the catheter 100 is rotated around its longitudinal axis, or in an alternative embodiment of the catheter, when the catheter is pushed forward in distal direction, over the helical segment 2103.

More generally, the catheter 100 may be configured to cooperate with a tissue piercing distal part, such as the helical section 2103, of the fourth elongated structure, wherein the catheter 100 may be configured to, when activated, cut tissue along a circumference of the inner lumen of the tubular structure of the catheter. The catheter may be configured to cooperate with a tissue piercing distal part of a fourth elongated structure, wherein the catheter may be configured to, when activated, cut tissue pierced by the fourth elongated structure along the circumference of the lumen of the fourth elongated structure.

The fourth elongated structure may comprise a first hollow tubular structure or tubular structure of a first material or composition which is connected to a helical geometry structure with a hollow core at its distal portion, possibly being made of a different second material or composition, but alternatively being made of the first material or composition. Both the first hollow tubular structure and the helical geometry structure may be flexible through their design and material. The fourth elongated structure may be reworked to increase flexibility, such as geometrical adjustments. The fourth elongated structure may be torsionally stiff. In certain embodiments, a diameter of a main body 2108 of the fourth elongated structure 2100 may have a smaller diameter than the diameter of the helical segment 2103. The helical segment 2103 may be torsionally stiff. The helix may have a certain winding height which can be, but not necessarily is, uniform along the length of the helical segment 2103. The helical segment 2103 can be used to pierce tissue if rotated in a first direction. The outer diameter of the helix may have a conical geometry. For example, the diameter at the distal end of the helical segment 2103 may be smaller than the diameter at the proximal end of the helical segment 2103. Alternatively, the outer diameter of the helical segment 2103 may have the same geometry throughout its length. For example, the diameter may be substantially constant from the most distal part to a more proximal part. The main body 2108 may have a helical segment 2104 itself, at the distal end of the main body 2108. A proximal portion of the helical segment 2103 may reciprocate with the helical segment 2104 of the main body. That way, the helical segment 2103 may be attached and fixed to the main body 2108. However, this is only an example of how the helical segment 2103 may be connected to the main body 2108. The helical segment 2103 may be connected or joined to the main body 2108 through another mechanical or chemical technique.

The main body 2108, which may be tubular, and the helical segment 2103, may be torsionally stiff by themselves and when combined so as to effect rotation of the distal tip by rotating a proximal portion of the main body 2108, while the fourth elongated structure 2100 is placed inside the catheter 100.

The main body 2108 and the helical section 2103 may be flexible to enable and retain steering capability of guiding devices, such as a guidewire or the third elongated structure 1400, 1500.

The helical structure 2103 may have an a-traumatic tip 2105 (blunt). In particular, the tip 2105 may be atraumatic if the fourth elongated structure 2100 is pushed in the distal direction along the longitudinal axis 2110, and/or if rotated around the longitudinal axis 2110 in the direction pointing towards the sharp tip 2102, in other words, opposite the orientation of the windings of the helix.

The helix 2107 may have the same inner diameter along its length. However, the helix 2107 may alternatively have differing diameters along the length.

The main body 2108 and helical section 2103 may comprise a lumen that can be used for guiding another device, or that allows the fourth elongated structure 2100 to be guided by another device towards a target position. A proximal part (not illustrated) of the fourth elongated structure 2100 may allow the user to rotate it clockwise or counter-clockwise freely. The proximal part (not illustrated) of the fourth elongated structure 2100 may be capable of being pushed or pulled for distal force application of the connected helix 2107 to a tissue. The rotation and/or insertion of the fourth elongated structure 2100 may, for example, be performed mechanically / manually, or, may be actuated by a motor on the proximal side. The fourth elongated structure 2100 may facilitate application of a vacuum device for applying suction to the lumen of the main body 2108 from a proximal position. A distal part of the helical structure 2107 may be trackable through being fluoroscopically opaque or containing fluoroscopically opaque markers. A distal part of the helical structure 2107 may be trackable through being integrated with an electromagnetic sensor, or, electromagnetically trackable. The fourth elongated structure 2100 may be designed such that it can be incorporated in a secondary tubular hollow structure (component or device) that has a lumen with a diameter greater than the outer diameter of the fourth elongated structure 2100.

Where markers may be attached to the fourth elongated structure 2100 proximally for being able to approximate component insertion relative to a secondary component or device.

An embodiment of the first elongated structure comprises a detector, such as a camera or an ultrasound probe or a combination thereof, at a distal end of the elongated structure, wherein the elongated structure has a substantially cylindrical shape with a circle segment as a base.

An embodiment of the second elongated structure comprises a lumen extending from a proximal end of the second endoluminal elongated structure to a distal end of the second endoluminal elongated structure, wherein the second elongated structure has a substantially cylindrical shape with a circle segment as a base.

An embodiment of the first endoluminal elongated structure comprises a detector, such as a camera, an optic fiber, or an ultrasound probe or a combination thereof, at a distal end of the elongated structure, and a connector near the distal end of the first elongated structure for attaching a second elongated structure to the distal end of the first elongated structure.

An embodiment of the second endoluminal elongated structure comprises a lumen extending from a proximal end of the second elongated structure to a distal end of the second endoluminal elongated structure, the lumen being suitable to allow passage of a third elongated structure, and a connector near the distal end of the second elongated structure for attaching a first elongated structure to the distal end of the second elongated structure.

Preferably, the circle segment of the first elongated structure and the circle segment of the second elongated structure match in the sense that when put together, the circle segments form a complete circle.

It will be understood that the combination of the catheter and other elongated structures disclosed herein is able to act as a functional device. It will be understood that the catheter, first elongated structure, second elongated structure, third elongated structure, and fourth elongated structure may be separate objects that can be packaged separately and that can be put together before and/or during use, by means of the connector and lumen(s) described throughout this disclosure. Possibly, several components can also act as a separate device when used in conjunction with other endoscopic devices, such as available catheters, extended working channels, robotic endoscopes.

According to another aspect of the invention, a method of exploring a lumen is provided, comprising
inserting a catheter into the lumen to be explored, wherein a material of a segment of the catheter is more flexible with respect to bending than a remainder of the catheter;
inserting a first elongated structure and a second elongated structure into a lumen of the catheter, wherein a distal end of the first elongated structure is fixed to a distal end of the second elongated structure;
guiding the catheter, the first elongated structure, and the second elongated structure by exerting a force in longitudinal direction to a proximal end of the catheter, first elongated structure, and second elongated structure; and
bending the distal end of the catheter by moving the first elongated structure in longitudinal direction with respect to the second elongated structure, by exerting a force in longitudinal direction to the first elongated structure or the second elongated structure at the proximal end of the catheter.

This method may facilitate navigating and manipulating networks of complex narrow channels.

The method may further comprise receiving an at least two-dimensional image from a detector at the distal end of the first elongated structure when the distal end of the first elongated structure is within the lumen to be explored.

The method may further comprise actuating a needle disposed inside the second elongated element to move the needle at least partly out of the second elongated element to puncture a tissue outside of the catheter;
moving a third elongated element through a lumen in the second elongated element into a lumen in the needle;
retracting the first elongated structure and the second elongated structure from the catheter while leaving the third elongated structure.

This may help to anchor the third elongated structure to the tissue that was punctured by the needle.

The method may further comprise inserting a fourth elongated structure having a tool at a distal end of the fourth elongated structure into the catheter, possibly guided by the third elongated structure, and applying the tool at the distal end of the catheter, near the distal end of the third elongated structure.

This may help to apply a tool at a well-defined location within a lumen.

The fourth elongated structure may have a lumen extending from a proximal end of the fourth elongated structure to the distal end of the fourth elongated structure. Before the fourth elongated structure is inserted into the catheter, the proximal end of the third elongated structure may be inserted into the lumen of the fourth elongated structure at a distal end of the fourth elongated structure, so that the fourth elongated element contains at least part of the third elongated structure. This may help to guide to fourth elongated structure to the distal end of the third elongated structure.

The tool of the fourth elongated structure may comprise a helical cutter, and the method may further comprise after the fourth elongated structure has been inserted into the catheter up to where the third elongated structure is anchored in the tissue, piercing the helical cutter into the tissue by rotating the helical cutter around its longitudinal axis, and cutting the tissue around the helical cutter by moving a cutting edge at a distal end of the catheter around the helical cutter.

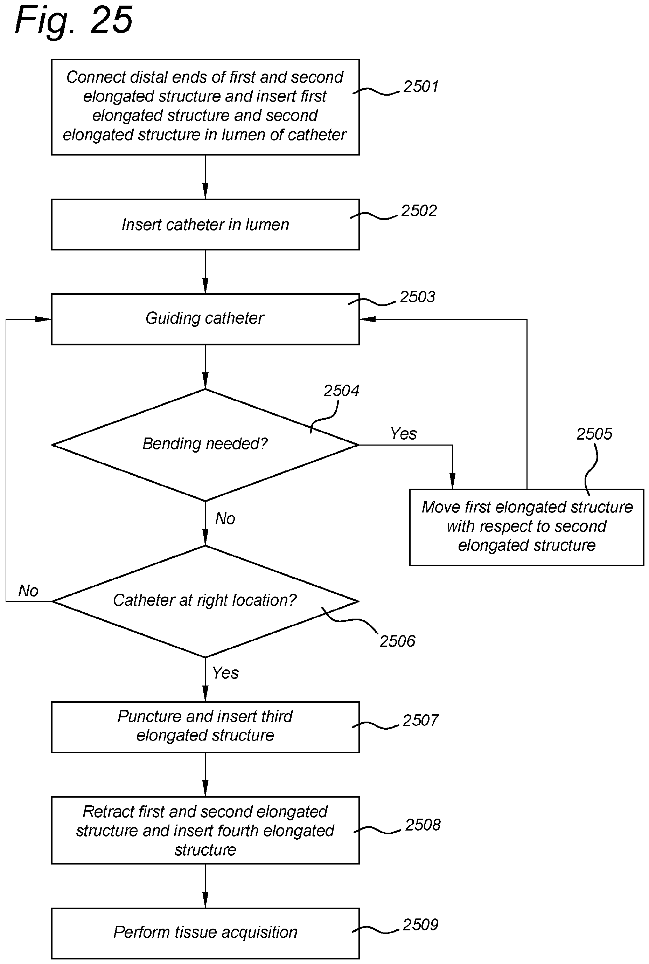

Fig. 25 shows a flowchart illustrating a method of exploring a lumen, and taking a biopsy. Although a biopsy is taken in the illustrated method by means of example, the method may be easily modified to perform any desired action using a suitable tool at a desired location instead of taking a biopsy.

The method may start with pre-procedural preparations in step 2501. The distal ends of a first elongated structure and a second elongated structure are connected, using a connector provided on either or both of these elongated structures. The first elongated structure may, for example, be a video-endoscopic device or an endobronchial ultrasound device or a combination thereof. The second elongated structure may have a lumen and optionally a puncturing device such as an actuable needle. It is also possible to prepare two sets of first and second elongated structure. The first set with a first elongated structure having a video-endoscope, and the second set with a first elongated structure having an ultrasound device. This allows to exchange the sets during the procedure while keeping the catheter inside the body.

The first and second elongated structure (attached to each other at their distal ends) may be inserted into a catheter, so that the first and second elongated structure go through a segment of the catheter that is more flexible than the remainder of the catheter. The first and second elongated structure may be inserted far enough so that the connector is distal to at least a portion of the flexible segment, however, this is not a limitation.

In certain embodiments, a conventional endoscope may be provided into the patient. This is however not mandatory.

In step 2502, the catheter with the first and second elongated structure disposed therein is inserted into a lumen of a body.

In case the conventional endoscope is provided in the patient, the catheter with the first and second elongated structure is inserted into a lumen of the conventional endoscope, up to and beyond a distal end of the conventional endoscope. This way, the conventional endoscope can be used for initial quick guidance through e.g. the large bronchia or vessels, and thereafter the catheter is protruded from a distal end of the conventional endoscope, to perform guided navigation through the narrower peripheral bronchia or vessels.

In step 2503, using video imaging (or ultrasound imaging) of the first elongated structure, one can navigate to the outer peripherals of the segment near to a lesion. In step 2504, it is decided whether it is necessary to flex the distal end of the catheter. If yes, then in step 2505, flexion of the catheter is achieved by pulling/pushing of the second elongated structure relative to the first elongated structure (and relative to the catheter).

Otherwise, it is decided in step 2506 whether the distal end of the catheter has reached the desired target location. If not, the guiding of the catheter is continued in step 2503. If the target location has been reached, in step 2507, the tissue may be punctured using a puncturing device of the second elongated structure, for example, under video-endoscopic guidance by the first elongated structure. Alternatively, one may also decide to deploy a different tool without using a puncturing device receding in the first elongated structure. This can for example be a tool such as the fourth elongated structure, or, tools as known to those familiar to the field in view of the present disclosure.

If the lesion is not easy to discern on video-endoscopic images, the first and second elongated structure may be retracted and replaced with the set of first and second elongated structure having the ultrasound probe. Then, the puncture can be done under ultrasonic visualization guidance.

With the needle or puncturing device at least partly in the punctured tissue, a third elongated structure may be inserted through a lumen in the second elongated structure into a lumen of the needle or puncturing device. The needle can be actuated to retract back into the second elongated structure.

After that, in step 2508, the first and second elongated structure may be removed from the catheter, leaving the catheter and the third elongated structure behind. This way, some space is freed up inside the catheter. This space may be utilized by inserting a fourth elongated structure into the catheter. If the fourth elongated structure has a lumen, the other tool may be guided by the third elongated structure as well. The fourth elongated structure may comprise any suitable tool at its distal end. Markings at the proximal side of the catheter, the third elongated structure, and/or the fourth elongated structure, may show when the distal tip of the fourth elongated structure is aligned with the distal tip of the third elongated structure and/or the distal tip of the catheter. The fourth elongated structure may comprise, for example, any type of biopsy tool or another interventional tool, such as an ablation tool.

For example, if the fourth elongated structure comprises a helical segment, as described hereinabove, the fourth elongated structure may be utilized by, after proper alignment of the distal tips, rotating the fourth elongated structure around its longitudinal axis. This way, the fourth elongated structure may anchor itself into the tissue, optionally being guided by the third elongated structure. Optionally, the third elongated structure may be pulled back slightly if needed (e.g. for increasing tissue specimen size). Optionally, the fourth elongated structure may be guided by means of optional sensor technology near the distal tip of the third elongated structure, or, external imaging techniques such as fluoroscopy or cone-beam CT imaging. Other tools may be used to fixate a portion of tissue instead of the helical tool.

Having anchored a part of tissue using the fourth elongated structure, the tissue fixated to and/or residing within the fourth elongated structure may be removed by pulling the fourth elongated structure in proximal direction. Before that, the tissue may be cut around the fourth elongated structure, for example by moving the catheter over the distal segment of the fourth elongated structure, thereby cutting the tissue around the fourth elongated structure. The catheter may cooperate with the fourth elongated structure in a cutting action based on, for example, a shearing strain. To that end, the catheter may contain a sharp edge. In certain embodiments, the sharp edge, or blade, may be directed in a transverse direction so that the cutting is facilitated if the catheter is rotated in a predefined direction around its longitudinal axis. Alternatively, the catheter may be pushed over the distal segment of the fourth elongated structure. With the proper dimensions of the fourth elongated structure and the catheter, this will cut the tissue in a cooperative action.

For example, when the distal tip of the catheter has reached the distal tip of the distal segment of the fourth elongated structure, the catheter may be kept in place while the fourth elongated structure is completely removed from the catheter through the proximal end of the catheter. Multiple biopsies may be taken by repeated application of the fourth elongated structure.

The tissue can be removed from the fourth elongated structure, e.g. from the helix, by using a tissue specimen removal tool. Alternative biopsy tools or treatment tools as known to skilled person can be used in conjunction with the catheter, first, second, and/or third elongated structures. This does not necessarily have to be preceded by usage of the helical tool.

Certain embodiments may provide an (actively) steerable endoscope which can navigate to relatively narrow peripherals of the lung under direct video-endoscopic vision, whilst simultaneously being able to provide a lumen size for secondary tools, such as the here presented incorporation of components. Certain embodiments may allow for (active) steering of the endoscope through a combination of separately provided components, as disclosed herein. These separate components may further each have a functionality in addition to steering the endoscope.

Certain embodiments may provide a relatively large lumen for secondary tools after navigation.

Certain embodiments may allow re-usability of high fidelity technology whilst only simpler components need be replaced (disposable) due to possible cleaning issues and/or mechanical fatigue or other issues.

Certain embodiments may allow for the use of a novel biopsy device, which is able to obtain a solid core of target tissue that is bigger than possible with any other biopsy device of its size. This may improve diagnostic accuracy. This biopsy device furthermore may operate in a novel way, whereby its forces for tissue acquisition remain within the device lumen and/or can be used for device steering.

Certain embodiments may provide simultaneously local real-time multi-modality based imaging verification of target tissue puncture (e.g. under local video-endoscopic and/or ultrasonic guidance).

Certain embodiments allow an intuitive combined workflow through smart integration of components and technology. By the design features disclosed herein, the physician may be enabled to intuitively combine navigation to confined spaces, that were previously unreachable, using diagnostic tools and treatment tools, that were previously unusable, due to size limitations.

Certain embodiments may allow a reduction of the complexity of the procedural workflow. Physicians may not have to decide about the to-be-used technology or screen to be assessed. Rather, the design allows for intuitive and simplified workflow, decreasing learning curve and/or increasing overall procedure performance.

Certain embodiments comprise a medical system comprising a catheter of a flexible tubular structure with a lumen extending in a longitudinal direction through the tubular structure of the catheter, wherein a distal segment of the catheter is more flexible than a remainder of the catheter. The medical system further comprises a first elongated structure and a second elongate structure being connected at a distal proportion by a connecting element. The first elongated structure and the second elongated structure have a cross section configured to together removably fit through the lumen of the catheter. The first elongated structure and the second elongated structure are movable with respect to each other at a proximal end in at least a longitudinal direction, except where they are fixed at the distal end of the first elongated structure and second elongated structure. Longitudinal movement of one structure with respect to the other results in bending of the catheter at the more flexible proportion. The first and second elongated structure have further individual properties that allow the medical system to be usable for intraluminal imaging and interventions.

Some or all aspects of the invention may be suitable for being to cooperate or be integrated with a form of software or external imaging, in particular a computer program product and/or X-ray based imaging and/or electromagnetic navigation technology based imaging. The computer program product may comprise a computer program stored on a non-transitory computer-readable media. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be executable by one or more processors.

The examples and embodiments described herein serve to illustrate rather than limit the invention. The person skilled in the art will be able to design alternative embodiments without departing from the spirit and scope of the present disclosure, as defined by the appended claims and their equivalents. Reference signs placed in parentheses in the claims shall not be interpreted to limit the scope of the claims. Items described as separate entities in the claims or the description may be implemented as a single hardware or software item combining the features of the items described.

## Claims

1. A medical system, comprising
a catheter (100) comprising a flexible tubular structure, the catheter having a lumen (106) extending in longitudinal direction through the catheter (100), wherein a material of a segment (102) of the catheter is more flexible with respect to bending than a remainder of the catheter;
a first elongated structure (600) and a second elongated structure (900);
a connector (601, 901) for attaching the first elongated structure (600) to the second elongated structure (900) near the distal end (605) of the first elongated structure (600) and the distal end (905) of the second elongated element (900);
the first elongated structure (600) and the second elongated structure (900) having a cross section (608, 907) configured to together removably fit through the lumen (106) of the catheter (100);
a proximal portion of the first elongated structure (600) up to the connector (601) and a proximal portion of the second elongated structure (900) up to the connector (901) being movable with respect to each other at least in a longitudinal direction of the first elongated structure (600) and the second elongated structure (900).

2. The medical system of claim 1, wherein the second elongated structure (900) comprises a lumen (906) extending from a proximal end of the second elongated structure (900) to the distal end (905) of the second elongated structure (900), the lumen (906) being suitable to allow passage of a third elongated structure (1400, 1500).

3. The medical system of claim 2, wherein the first elongated structure (600) comprises a detector (606) at its distal end, wherein the detector (606) comprises for example a camera, an optic fiber, an ultrasound imaging probe, or a radiation monitoring probe.

4. The medical system of any preceding claim, wherein the connector (601, 901) is configured for detachably fixing the distal end (605) of the first elongated structure (600) to the distal end (905) of the second elongated structure (900).

5. The medical system of any preceding claim, wherein the second elongated structure (900) further comprises a needle (1201) that is configured to be actuated proximally and, when actuated, move at least partially out of the distal end (905) of the second elongated structure (900) to puncture a tissue.

6. The medical system of any one of claims 2 to 5, further comprising the third elongated structure (1400, 1500), the third elongated structure (1400, 1500) having a cross section configured to fit through the lumen (906) of the second elongated structure (900) and optionally through a lumen (1206) of a needle (1201) at a distal end (605, 905) of the first or second elongated structure (600, 900).

7. The medical system of any preceding claim, wherein the catheter (100) is configured to cooperate with a tissue piercing distal part (2103) of a fourth elongated structure (2100), wherein the catheter (100) is configured to, when activated, cut tissue along a circumference of the lumen (106) of the catheter (100).

8. The medical system of claim 7, further comprising the fourth elongated structure (2100), wherein the fourth elongated structure (2100) has a cross section (2109) configured to fit through the lumen (106) of the catheter (100) around the third elongated structure (1400, 1500).

9. The medical system of claim 1, wherein the first elongated structure (600) has a first cross section (608) and the second elongated structure (900) has a second cross section (907) with an edge (909) that matches a corresponding edge (609) of the first cross section (608), wherein when the first elongated structure (600) and the second elongated structure (900) are joined at their matching edges (609, 909) the cross section of the combination of the first elongated structure (600) and the second elongated structure (900) have an outline that corresponds to an outline of a cross section of the lumen (106) of the catheter (100).

10. The medical system of claim 1 or 9, wherein the cross section (608) of the first elongated structure (600) is a circle segment and the cross section (907) of the second elongated structure (900) is a circle segment.

11. The medical system of any preceding claim, wherein the more flexible segment (102) of the catheter (100) is inclined to bend in a predetermined direction and/or wherein the more flexible segment (102) of the catheter (100) is located at the distal end (105) of the catheter (100).

12. The medical system of any preceding claim, further comprising
an actuator arranged at a proximal end of the tubular structure of the catheter (100) and configured to control longitudinal (104) movement of at least one of the first elongated structure (600) and the second elongated structure (900) with respect to the other one of the first elongated structure (600) and the second elongated structure (900), to achieve bending of the catheter (100) at the flexible segment (102).

13. An endoscope comprising a first endoluminal elongated structure comprising
a detector (606, 1706), such as a camera, an optic fiber, or an ultrasound probe, at a distal end (605) of the first elongated structure (600, 1700); and
a connector (601) near the distal end (605) of the first elongated structure (600, 1700) for attaching a second elongated structure (900) to the distal end (600, 1700) of the first elongated structure.

14. A second endoluminal elongated structure comprising
a lumen (906) extending from a proximal end of the second elongated structure (900, 1200) to a distal end (905) of the second endoluminal elongated structure (900, 1200), the lumen (906) being suitable to allow passage of a third elongated structure (1400, 1500); and
a connector (901) near the distal end (905) of the second elongated structure (600, 1200) for attaching a first elongated structure (600, 1700) to the distal end (905) of the second elongated structure (900, 1200).
